# EUROPEAN PATENT SPECIFICATION

(11) **EP 4 419 184 B1**
(45) Date of publication and mention of the grant of the patent: **11.11.2026**
(21) Application number: 22869563.1
(22) Date of filing: 19.09.2022
(51) Int. Cl.: A61M 25/01

(54) **CATHETER**
KATHETER
CATHÉTER

(30) Priority: 18.09.2021 IN 202141042319
(43) Date of publication of application: 28.08.2024
(73) Proprietor: Kalyanasundaram, Arun, Tamil Nadu 600 041 (IN)
(72) Inventor: Kalyanasundaram, Arun, Tamil Nadu 600 041 (IN)
(74) Representative: Lavoix
(86) International application number: PCT/IN2022/050834
(87) International publication number: WO 2023/042227

(56) References cited:
- EP-A1- 4 289 463
- EP-B1- 3 758 781
- WO-A1-2020/206101
- WO-A1-90/07352
- US-A1- 2018 361 114
- US-A1- 2019 314 077
- US-A1- 2020 246 596
- US-B1- 7 048 719

## Description

### TECHNICAL FIELD

The present invention relates, generally, to medical devices and, particularly but not exclusively, to catheters for assisting medical procedures inside a lumen such as blood vessels or ducts thereof.

### BACKGROUND

A catheter is a medical device of hollow thin-walled tubular structure that is inserted in a lumen or duct of a human or animal body through a natural access site, or a temporary artificial access site created by a physician. The access site provides access to the physician to insert the catheter and perform an interventional procedure or treat a disease. By modifying the material and design, catheters can be customized to meet different requirements for cardiovascular, urological, gastrointestinal, neurovascular, and ophthalmic procedures.

In cardiovascular interventional procedures, different types of catheters are used for a variety of tasks. Guide catheters are primarily used for creating an access path from the access site and through the lumen for insertion of a guidewire or interventional / diagnostic devices. The guidewires usually cross entire length of the guide catheters and goes further till a location where treatment is required. Guide catheters need to be stiffer to provide support for an implant delivery catheter advancement into the lumen or duct such as a coronary artery. Guide catheters usually have large lumens and thinner walls but at the same time they should be kink resistant, create less friction and have good torque control. In addition, an atraumatic soft tip is also needed in an effective guide catheter. Further, there are sub-categories under broad category of guide catheters and micro catheter is one such category. Micro catheters are thin walled, small diameter catheters used in minimally invasive applications where the lumen diameter is very small. These catheters are utilized in interventional cardiology for the purpose of exchanging wires or providing dedicated access paths, partially or completely, to particular guidewires during minimally invasive procedures when arteries are completely or almost completely blocked (CTO: Chronic Total Occlusion), in antegrade approach, in retrograde approach or for crossing collateral arteries. Mostly, microcatheters have atraumatic soft tips, optionally radiopaque.

Further, in some procedures involving guide catheters, specifically micro catheters, multiple guidewires are needed for performing parallel or sequential procedural steps. For such procedures, dual or multiple lumen catheters are utilized for accessing arteries, branches originating from main arteries, collateral arteries etc. and for a plethora of other specific coronary or endovascular conditions. The advantage of a dual or multiple lumen catheter is that wire access in the parent vessel is not lost. One another example of using multiple guidewires is "Parallel wire technique" where the first wire is subintimal or inside the body of the chronic total occlusion while the second wire is crossed into the true lumen. However, two or more than two lumens within a main lumen of the catheter increases the overall diameter of the main lumen of a guide catheter or specifically of a micro catheter thus limiting the applicability of the catheter to narrow lumens and ducts in human or animal body.

Conventionally, to meet the different requirements, the guide catheters have different constructions, designs, and properties each of which attempt to address different properties, or a combination of the properties as mentioned above. However, due to design compromises, most of the guide catheters meet only limited no. of properties and objectives outlined above, resulting in restricted utility and effectiveness. Documents US20200246596A1, EP3758781B1, EP4289463A1, WO2020206101A1 and US20180361114A1 disclose each catheter of the prior art.

Therefore, there is still a need for a guide catheter to avoid the shortcomings known in the art and specifically a micro catheter that gives better and accurate accessibility. Also, such micro catheters help in deployment of trans-vascularly deliverable prosthetic implants.

### SUMMARY OF THE INVENTION

Described herein is a medical device, specifically a catheter, more specifically a micro catheter, for accessing the arteries. The subject technology, according to various aspects, is summarized below.

According to the invention, a catheter according to claim 1 is provided . Optional aspects of the invention are defined in the dependant claims. The second exit port and the first entry port are situated at an angle to each other which preferably is 180° to each other. In addition, the second exit port and the first entry port are situated at a distance with each other longitudinally. Optionally, these two ports are situated on same length longitudinally but at 180° to each other.

According to the invention, the catheter has a single lumen, extending between a luer at proximal end and a tip at distal end, and the peripheral surface of the catheter comprises a first entry port, at least one second exit port, a first exit port at the tip for a first guide wire that enter through the first entry port and exit from the first exit port and a second entry port situated at the luer. The at least one second exit port aids a second guidewire that enter through the second entry port and exit from the second exit port. According to an embodiment, the lumen has two second exit ports situated on the peripheral surface of the lumen at an angle to each other which preferably situated at 180° to each other. In addition, the two second exit ports include a primary second exit port and a secondary second exit port, the primary second exit port and the secondary second exit port are situated at distance with each other longitudinally. Optionally, these two second exit ports are situated on same length longitudinally but at 180° to each other. The first entry port is located between the tip and the two second exit ports.

According to a further embodiment of the invention, the lumen comprises an expandable balloon connected near the distal end of the catheter wherein the expandable balloon is not in fluid communication with the lumen, but the expandable balloon is connected with distal end of an inflation lumen. The proximal end of the inflation lumen is connected to an inflation port situated on the luer. In addition, each entry port or exit port situated on the periphery of the catheter comprises a plurality of joints for enabling an insulated passage across the inflation lumen for guiding of the guidewires between the single lumen and outside of the catheter

According to yet a further embodiment of the invention, the lumen of the catheter is divided in two lumens, a first lumen part and a second lumen part, by a dividing wall situated longitudinally inside the lumen. In addition, a third entry port is provided on the luer. This dividing wall provides separate lumen for the guidewires entering from the second entry port and the third entry port.

The above aspects are further illustrated in the figures and described in the corresponding description below. It should be noted that the description and figures merely illustrate principles of the present invention. Therefore, various arrangements that encompass the principles of the present invention, although not explicitly described or shown herein, may be devised from the description.

### BRIEF DESCRIPTION OF ACCOMPANYING DRAWINGS

The detailed description is described with reference to the accompanying figures.

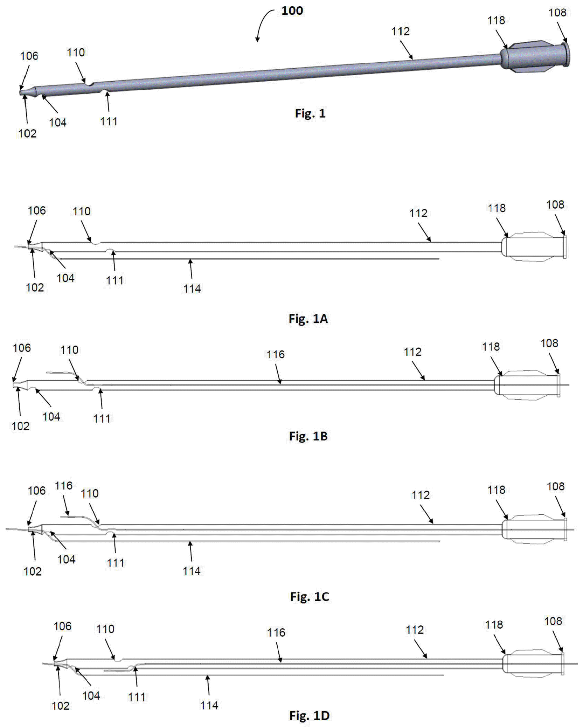

FIG. 1 illustrates a three-dimensional side view of a micro catheter, according to an embodiment of the present invention.
FIG. 1A illustrates a two-dimensional longitudinal cross-sectional, side view of a micro catheter showing a first guidewire entering a first entry port and exiting from first entry port, according to an embodiment of the present invention.
FIG. IB illustrates a two-dimensional longitudinal cross-sectional, side view of a micro catheter showing a second guidewire entering through a second entry port and exiting from a second exit port, according to an embodiment of the present invention.
FIG. 1C and ID illustrates a two-dimensional longitudinal cross-sectional, side view of a micro catheter showing a first guidewire entering a first entry port and exiting from first entry port and a second guidewire entering through a second entry port and exiting either from a primary second exit port and a secondary second exit port, according to an embodiment of the present invention.

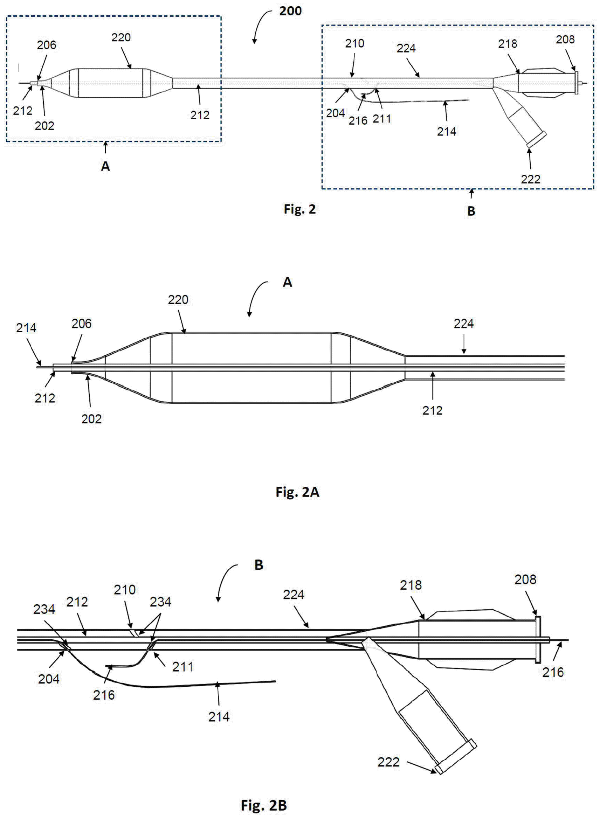

FIG. 2 illustrates a two-dimensional longitudinal cross-sectional, side view of a micro catheter showing an expandable balloon on distal end of the catheter and showing a second guidewire entering through a second entry port and exiting from a secondary second exit port and a first guidewire entering a first entry port and exiting from first entry port, according to an embodiment of the present invention.
FIG.2A illustrates a two-dimensional longitudinal cross-sectional, side view of section 'A' of a micro catheter showing the expandable balloon on distal end of the catheter and showing a first guidewire entering a first entry port and exiting from first entry port, according to an embodiment of the present invention.
FIG. 2B illustrates a two-dimensional longitudinal cross-sectional, side view of section 'B' of a micro catheter showing the proximal end of the catheter showing luer, inflation port, a first entry port, a primary second exit port, a secondary second exit port, a plurality of joints, a first guidewire and a second guidewire, according to an embodiment of the present invention.

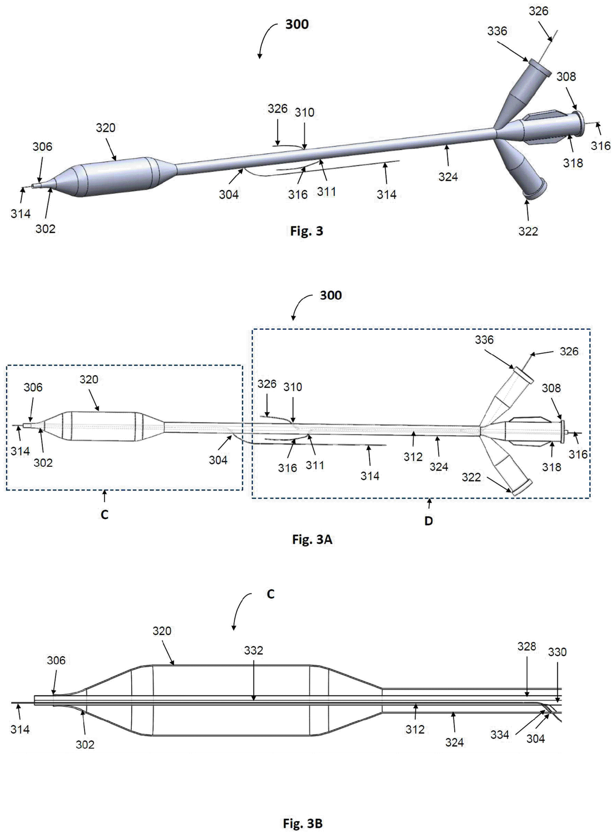

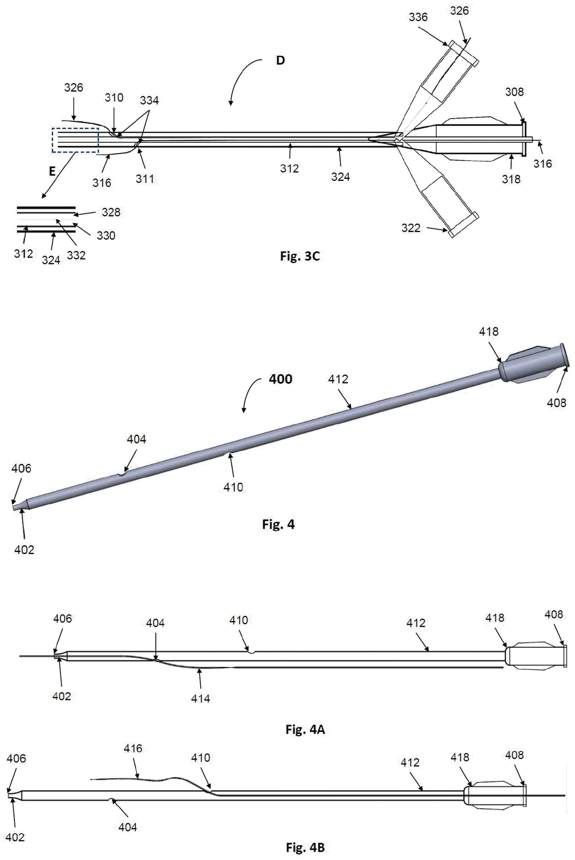

FIG. 3 illustrates a three-dimensional side view of a micro catheter showing an expandable balloon on distal end of the catheter and showing luer, inflation port, a first entry port, a second entry port, a third entry port, a primary second exit port, a secondary second exit port, a third exit port, a first guidewire, a second guidewire, a third guidewire, according to an embodiment of the present invention.
FIG. 3A illustrates a two-dimensional longitudinal cross-sectional, side view of a micro catheter showing an expandable balloon on distal end of the catheter and showing luer, inflation port, a first entry port, a second entry port, a third entry port, a primary second exit port, a secondary second exit port, a third exit port, a first guidewire, a second guidewire, a third guidewire, according to an embodiment of the present invention.
FIG. 3B illustrates a two-dimensional longitudinal cross-sectional, side view of section 'C' of a micro catheter showing an expandable balloon on distal end of the catheter, a lumen divided by a dividing wall into a first lumen part and a second lumen part, a first guidewire entering in the second lumen through a first entry port and exiting from a first exit port, according to an embodiment of the present invention.
FIG. 3C illustrates a two-dimensional longitudinal cross-sectional, side view of section 'D' of a micro catheter showing proximal end of the catheter showing luer, a dividing wall, a first lumen part, a second lumen part, inflation port, inflation lumen, a second entry port, a third entry point, a primary second exit port, a secondary second exit port, a plurality of joints, a second guide wire and a third guidewire, according to an embodiment of the present invention.
FIG. 4 illustrates a three-dimensional side view of a micro catheter, according to an embodiment of the present invention.
FIG. 4A illustrates a two-dimensional longitudinal cross-sectional, side view of a micro catheter showing a first guidewire entering a first entry port and exiting from first entry port, according to an embodiment of the present invention.
FIG. 4B illustrates a two-dimensional longitudinal cross-sectional, side view of a micro catheter showing a second guidewire entering through a second entry port and exiting from a second exit port, according to an embodiment of the present invention.

### DESCRIPTION OF THE INVENTION

In the following description, for purpose of explanation, specific details are set forth in order to provide an understanding of the present disclosure. It will be apparent, however, to one skilled in the art that the present disclosure may be practiced without these details. One skilled in the art will recognize that embodiments of the present disclosure, one of which is described below, may be incorporated into a number of systems. Further, structures and devices shown in the figures are illustrative of exemplary embodiment of the present disclosure and are meant to avoid obscuring the present disclosure.

Present disclosure explains an embodiment of a catheter, specifically a micro catheter, that is designed to have a low profile (small diameter) lumen that has multiple ports on its peripheral surface to facilitate entry and exit of different guidewires. The lumen and the ports are designed in such a way so that the guidewires do not come in physical contact with each other. The small profile of the catheter makes access to narrow arteries easier whereas the multiple entry-exit ports allow using the same single lumen for one and more than one guidewire that helps in complex medical procedures e. g. antegrade fenestration re-entry procedure. Thus, comparing with the traditional multiple lumen micro catheters, embodiments of the present disclosure explain a micro catheter providing better utility.

According to an embodiment of the present disclosure, the medical device is a catheter, more specifically a micro catheter, having a single lumen with a plurality of ports on its peripheral surface, in addition to a luer at the proximal end and a tip at the distal end. A plurality of ports includes at least a first entry port and a second exit port. The luer also houses a second entry port and the tip houses a first exit port. Out of the plurality of the ports on the periphery of the catheter, at least two ports are situated at an angle to each other in circumferential direction e. g. the second exit port and the first entry port. The angle between these two ports may vary from 10° to 180°. However, more preferably, the angle is selected from 30°, 45°, 60°, 90°, 120°, 145°, 150° and 180°. More preferably, the angle is selected from 90° and 180°.

According to another embodiment of the present disclosure, the medical device is a catheter having a single lumen extending between a luer at a proximal end and a tip at a distal end. A first entry port is situated on the peripheral surface of the lumen and a first exit port at the tip for a first guidewire that enter through the first entry port and exit from the first exit port. A second entry port is situated at the luer and at least one second exit port on the peripheral surface of the lumen for a second guidewire that enter through the second entry port and exit from the second exit port. According to the embodiment, a plurality of ports is located on its peripheral surface, in addition to a luer at the proximal end and a tip at the distal end. A plurality of ports includes a first entry port, a primary second exit port and a secondary second exit port. The luer also houses a second entry port and the tip houses a first exit port. Out of the plurality of the ports on the periphery of the catheter, at least two ports are situated at an angle to each other in circumferential direction e. g. the primary second exit port and the secondary second exit port. The angle between these two ports may vary from 10° to 180°. However, more preferably, the angle is selected from 30°, 45°, 60°, 90°, 120°, 145°, 150° and 180°. More preferably, the angle is selected from 90° and 180°.

In addition, these two ports, e. g. the primary second exit port and the secondary second exit port, are also situated at distance with each other in longitudinal direction. Optionally, these two ports are situated on same length in longitudinal direction. Further, at least one another port, the first entry port, is also situated on periphery of the catheter and closer to the tip of the catheter comparing to the remaining ports. The tip of the catheter is atraumatic and connected to the lumen. An operator introduces a first guidewire through the first entry point and the first guidewire travels through a part of the lumen, the tip and exits from the first exit port. Process-wise, in parallel or in series, the operator introduces a second guidewire through the second entry point in the luer and the second guidewire travels through a part of the lumen and exits either from the second primary exit port or the secondary second exit port where these ports situated at an angle with each other in circumferential direction as well as at a distance with each other in longitudinal direction. One preferred angle is 180° but it can be varied depending on the application. Preferred distance is 1 mm, but it can be varied depending on the application. Further, the first entry port and the second exit port can be in alignment in longitudinal direction but not necessarily need to be.

The single lumen provides travel path for the first guidewire as well as the second guidewire. One such path initiates from the second entry port at the luer till either the primary second exit port or the secondary second exit port. Another such path initiates from the first entry port on the periphery of the catheter and first guidewire travels till the first exit port. Both the travel paths are exclusive to each other i.e., the first guidewire and the second guidewire never contact each other physically, hence, there is no possibility of entanglement. Also, this helps in keeping the overall profile of the lumen smaller and the smaller profile helps in accessing narrow arteries.

In addition, the medical device explained herein is, in some ways, is a hybrid of an OTW (over the wire) catheter and a Rx port type catheter. The catheter part from the second entry port till the primary second exit port or the secondary second exit port is similar to a OTW catheter whereas the catheter part from the first entry port till the first exit port is similar to a Rx port type catheter. Hence, the micro catheter explained here provides advantages of both type of catheters by optimizing the location of the first entry port, the primary second exit port, the secondary second exit port and the angle between the primary second exit port and the secondary second exit port.

According to another embodiment of the present disclosure, the catheter has an expandable balloon on distal part of the catheter. The balloon is expanded using any inflation liquid known in the art and the inflation liquid is injected into and inflation lumen through an inflation port situated on the luer. The inflation lumen is connected to the balloon. The expandable balloon is connected with distal end of the inflation lumen. In addition, in one design variation, concentric to the inflation lumen is the lumen extending from the luer till the tip of the catheter. The inflation lumen is around the lumen and the inflation liquid flows through the cross-sectional area between both the lumens. The first guidewire and the second guidewire travel through the lumen and as explained earlier, the lumen has a plurality of ports on its peripheral surface. Each port is connected to a joint that is extended to cross the inflation lumen in radial direction, either perpendicularly or at an angle with the longitudinal axis. A first entry port situated on the peripheral surface of the lumen and a first exit port at the tip for a first guidewire that enter through the first entry port and exit from the first exit port. A second entry port situated at the luer and a primary second exit port and a secondary second exit port situated on the peripheral surface of the lumen at an angle for a second guidewire that enters through the second entry port and exits either through the primary second exit port or through the secondary second exit port. The plurality of joints provides a passage, insulated from inflation liquid, for the guidewires i.e., first and second guide wires for them to come out of the peripheral surface of the inflation lumen or the catheter. The catheter is used for implantation of an implant.

According to yet another embodiment of the present disclosure, the expandable balloon on distal part of the catheter is expanded using the inflation liquid that can be injected through the inflation port situated on the luer and connected to the balloon through the inflation lumen. Further, inside, and concentric to the inflation lumen, is the lumen extending from the luer till the tip of the catheter and having a dividing wall inside the lumen dividing the cross-sectional area of the lumen in two parts thus providing two lumens inside the single lumen: a first lumen part and a second lumen part. The single lumen is divided by a dividing wall situated longitudinally inside the lumen. A third guide wire enters through a third entry port, situated on the luer, and travels through the first lumen part and exits from the second exit port i.e., primary second exit port. The second guidewire enters through a second entry port, situated on the luer, and travels through the second lumen and exits from the secondary second exit port. Each port is connected to a joint that is extended to cross the inflation lumen in radial direction, either perpendicularly or at an angle with the longitudinal axis. The plurality of joints provides a passage, insulated from inflation liquid, for the guidewires for them to come out of the peripheral surface of the inflation lumen or the catheter.

According to yet another embodiment of the present disclosure, the single lumen is not divided in two lumens across the length of the lumen. Instead, the dividing wall merges with the inner periphery of the lumen just ahead of the primary second exit port or the secondary second exit, whichever is towards the tip.

According to yet another embodiment of the present disclosure, at least one implantable device is crimped on the expandable balloon at the distal end of the catheter for therapeutic substance delivery or for deployment as an implant.

According to yet another embodiment of the present disclosure, the catheter may have a plurality of expandable balloons arranged in series and connected to the same inflation lumen.

According to yet another embodiment of the invention, at least one entry/exit port is situated either on main body of the balloon or in between any two expandable balloons, in case a plurality of expandable balloons are attached on the catheter.

According to yet another embodiment of the present disclosure, the shape of the expandable balloon is selected from cylindrical balloon with conical ends, oval shaped balloons, cutting balloon, dumb-bell shaped balloon, or branched balloon.

According to yet another embodiment of the present disclosure, the expandable balloon at the distal end of the catheter may have arrangements for therapeutic substance delivery or an implant for deployment.

During the procedure, due to specific spatial arrangement of the exit and entry ports, the guidewires do not interact with each other.

According to yet another embodiment of the present disclosure, different elements, all or selected ones, of the catheter are connected using adhesive, sealant, glue, threads, welding, other mechanical connection measures known in the art, other chemical connection measures known in the art or a combination thereof.

The methods of use that are described hereinafter do not fall under the scope of the invention as claimed.

According to yet another embodiment of the present disclosure, the catheter may be used in treating any abnormality or in any medical procedure related to heart, kidney, lever, brain, pancreas, lungs, digestive system, endovascular system, any tract, duct or any conduit in animal or human body. More specifically, the microcatheter can be deployed in an artery, vein, heart valves, oesophageal duct, bile duct, urinary tract, alimentary tract, tracheobronchial tree, cerebral aqueduct or genitourinary system of an animal or human body.

According to another embodiment of the present disclosure, the catheter may be used in implantation of an implant. The implant may be, but not limited to, a stent, a valve, a mesh, a balloon, a patch, a drug-containing matrix, a shunt, or a combination thereof.

The above aspects are further illustrated in the figures and described in the corresponding description below. It should be noted that the description and figures merely illustrate principles of the present invention. Therefore, various arrangements that encompass the principles of the present invention, although not explicitly described or shown herein, may be devised from the description.

Referring FIG. 1 that shows a three-dimensional side-view of a micro catheter (100) having a single lumen (112) with a plurality of ports (104, 111, 110) on its peripheral surface, in addition to a luer (118) at the proximal end and a tip (102) at the distal end. The ports include a first entry port (104), a primary second exit port (110) and a secondary second exit port (111). The luer (118) also houses a second entry port (108) and the tip (102) houses a first exit port (106). The primary second exit port (110) and the secondary second exit port (111) are situated at an angle of 180° to each other. The first entry port (104) is situated between the second exit port (110) and the tip (102).

Referring FIG. 1A to 1D which show two-dimensional longitudinal cross-sectional, side view of a catheter (100) where a first guidewire (114) enters through a first entry port (104) and exit from a first exit port (106). A second guidewire (116) enters through a second entry port (108) and exits either through a primary second exit port (110) or a secondary second exit port (111). There is no defined sequence to insert the guidewires. Also, at a particular moment, the catheter may house both the guidewires, either guidewire or no guidewire. However, both the guidewires do not contact each other, and different sections of the same lumen (112) are used as travel paths for the first guidewire (114) or the second guidewire (116).

Referring FIG. 2 that shows a two-dimensional longitudinal cross-sectional, side view of a catheter (200) showing an expandable balloon (220) on distal end of the catheter and showing a second guidewire (216) entering through a second entry port (208) situated on a luer (218) and exiting from a secondary second exit port (211) and a first guidewire (214) entering a first entry port (204) and exiting from a first exit port (206) situated on a tip (202). An inflation port (222) is also situated on the luer (218) and the inflation port (222) is connected to the inflation lumen (224). The inflation lumen (224) is connected to the expandable balloon (220). Further, the inflation lumen (224) is co-centric to the lumen (212).

FIG. 2A shows a magnified, two-dimensional longitudinal cross-sectional, side view of section 'A' showing distal end of a catheter (200). The illustration shows an expandable balloon (220) on distal end of the catheter, a first guidewire (214) exiting from a first exit port (206) situated on a tip (202). An inflation lumen (224) is connected to the expandable balloon (220) and concentric to the inflation lumen (224) is a lumen (212).

FIG. 2B shows a magnified, two-dimensional longitudinal cross-sectional, side view of section 'B' showing proximal end of a catheter (200). The illustration shows a luer (218), a second guidewire (216) entering through a second entry port (208) and exiting from a secondary second exit port (211). A first guidewire (214) is entering a first entry port (204). An inflation port (222) is also situated on the luer (218) and the inflation port (222) is connected to the inflation lumen (224). The inflation lumen (224) is co-centric to the lumen (212). Further, each entry or exit port (204, 210, 211) situated on the periphery of the lumen (212) comprises a plurality of joints (234) that provides an insulated passage across the inflation lumen (224) for travel of guidewires (214, 216) between the lumen (212) and outside the inflation lumen (224). The primary second exit port (210) and the secondary second exit port (211) are situated at an angle of 180° to each other and at a distance from each other longitudinally.

Referring FIG. 3 that shows a three-dimensional side-view of a catheter (300) showing an expandable balloon (320) on distal end of the catheter and showing a second guidewire (316) entering through a second entry port (308) situated on a luer (318) and exiting from a secondary second exit port (311) and a first guidewire (314) entering a first entry port (304) and exiting from a first exit port (306) situated on a tip (302). The illustration also shows a third entry port (336) and a third guidewire (326) that enters through the third entry port (336) and exits through a second exit port (310).

Referring FIG. 3A that shows a two-dimensional longitudinal cross-sectional, side view of a catheter (300) showing an expandable balloon (320) on distal end of the catheter and showing a second guidewire (316) entering through a second entry port (308) situated on a luer (318) and exiting from a secondary second exit port (311), a first guidewire (314) entering a first entry port (304) and exiting from a first exit port (306) situated on a tip (302), and a third guidewire (326) entering through a third entry port (336) situated on a luer (318) and exiting from an second exit port (310).

FIG. 3B shows a magnified, two-dimensional longitudinal cross-sectional, side view of section 'C' showing distal end of a catheter (300). The illustration shows an expandable balloon (320) on distal end of the catheter, a first guidewire (314) entering from a first entry port (304) and exiting from a first exit port (306) situated on a tip (302). An inflation lumen (324) is connected to the expandable balloon (320) and concentric to the inflation lumen (324) is a lumen (312). The lumen (312) is cross-sectionally divided in two lumens across the length of the lumen (312) by a dividing wall (332). Further, the first entry port (304) is situated on the periphery of the lumen (312) comprises a plurality of joints (334) that provides an insulated passage across the inflation lumen (324) for travel of a first guidewire (314) between the lumen (312) and outside the inflation lumen (324).

FIG. 3C shows a magnified, two-dimensional longitudinal cross-sectional, side view of section 'D' showing proximal end of a catheter (300). The illustration shows a luer (318), a second guidewire (316) entering through a second entry port (308) and exiting from a secondary second exit port (311). A third guidewire (326) is entering a third entry port (336) and exiting from a second exit port (310). An inflation port (322) is also situated on the luer (318) and the inflation port (322) is connected to the inflation lumen (324). The inflation lumen (324) is co-centric to the lumen (312). Further, each entry or exit port (310, 311) situated on the periphery of the lumen (312) comprises a plurality of joints (334) that provides an insulated passage across the inflation lumen (324) fortravel of guidewires (326, 316) between the lumen (312) and outside the inflation lumen (324). The second exit port (310) and the secondary second exit port (311) are situated at an angle of 180° to each other and at a distance from each other longitudinally. In addition, as a further magnified section 'D' shows a lumen (312) divided into two parts by a diving wall (332) and thus a first lumen part (328) and a second lumen (330) are formed inside the lumen (312). The first lumen part (328) provides a travel path for the third guidewire (326) and the second lumen provides a travel path to the second guidewire (316).

Referring FIG. 4 illustrates a three-dimensional side view of a micro catheter (400), according to an embodiment of the present invention. FIG. 4A and FIG. 4B illustrates a two-dimensional longitudinal cross-sectional, side view of a micro catheter (400) having a first guidewire (414), a second guidewire (416), a first guidewire entry port (404), a second guidewire entry port (408), a first guidewire exit port (406), a second guidewire exit port (410), a single lumen (412), a tip (402), and a handle. Figures 4A and 4B shows an embodiment where the guide catheter (400) has a single main lumen (412) that can accommodate the first guidewire (414) and the second guide wire (416). The main lumen (412) has the first guidewire entry port (404) on a peripheral surface of the single lumen (412) but after the second guidewire exit port (410), and towards the distal end of the guide catheter (400). The second guidewire entry port (408) is located on the handle towards the luer (418) proximal to an operator. The second guidewire exit port (410) exists on peripheral surface of the single lumen (412) opposite to the first entry port (404) at a distance. The first guidewire exit port (406) is located on the tip (402) of the guide catheter (400). The tip (402) is atraumatic and connected to the main lumen (412). The first guidewire (414) enters a first entry port (404) and exits from first entry port (406). The second guidewire (416) enters through the second entry port (408) and exits from the second exit port (410).

Overall, the catheter (100, 200, 300, 400) as described in the invention has low profile due to single main lumen that enhances its accessibility to the lumens, arteries or ducts of small diameters and of humans or animals. Further, the spatial arrangement of entry ports and exit ports for different guidewires provide utility of a multiple lumen catheter but with smaller profile and no interaction of guidewires. Further, specific positioning of exit and entry ports on side of the main lumen provide efficient manoeuvring to the physician.

In addition, in some embodiments, addition of an expandable balloon enhances the catheter's anchoring capability, and the expandable balloon can also be used for therapeutic substance delivery or in deployment of an implant. The overall profile of the catheter remains comparatively smaller due to lower profile of the lumen. Also, in case of a single lumen divided by a dividing wall, the distal end of the catheter remains a single lumen during the application and provides comparatively a smaller profile to access narrower arteries.

Although design and application of the catheter (100, 200, 300, 400) are described, it is to be understood that the present invention is not limited, within the scope of the claims, to the specific features or methods described. Rather, the specific features and methods are disclosed as implementations of the catheter (100, 200, 300, 400).

In the above description, for purpose of explanation, specific details are set forth in order to provide an understanding of the present disclosure. It will be apparent, however, to one skilled in the art that the present disclosure may be practiced without these details. One skilled in the art will recognize that embodiments of the present disclosure, one of which is described below, may be incorporated into a number of systems. Further, structures and devices shown in the figures are illustrative of exemplary embodiment of the present disclosure and are meant to avoid obscuring the present disclosure.

### List of Reference Numerals

| | |
|---|---|
| 100, 200, 300, 400 | Catheter |
| 102, 202, 302, 402 | Tip |
| 104, 204, 304, 404 | First entry port |
| 106, 206, 306, 406 | First exit port |
| 108, 208, 308, 408 | Second entry port |
| 110, 210, 310, 410 | Second exit port |
| 111, 211, 311 | Secondary second exit port |
| 112, 212, 312, 412 | Lumen |
| 114, 214, 314, 414 | First guidewire |
| 116, 216, 316, 416 | Second guidewire |
| 118, 218, 318, 418 | Luer |
| 220, 320 | Expandable Balloon |
| 222, 322 | Inflation port |
| 224, 324 | Inflation lumen |
| 336 | Third entry port |
| 326 | Third guidewire |
| 328 | First lumen part |
| 330 | Second lumen part |
| 332 | Dividing wall |
| 234, 334 | A plurality of joints |

## Claims

1. A catheter (100; 200; 300; 400) for accessing lumen, comprising:
- a single lumen (112; 212; 312; 412) for one and more than one guidewire, the said single lumen (112; 212; 312; 412) extending along a longitudinal axis between a luer (118; 218; 318; 418) at a proximal end and a tip (102; 202; 302; 402) at a distal end;
- a first entry port (104; 204; 304; 404), which is located on a peripheral surface of the single lumen (112;...; 412), and a first exit port (106; 206; 306; 406), which is located at the tip (102;...; 402) and which is provided for a first guidewire (114; 214; 314; 414) that enters through the first entry port (104;... ; 404) and exits from the first exit port (106;...; 406); and
- a second entry port (108; 208; 308; 408), which is located at the luer (118;... ;408), and at least one second exit port (110; 210; 310; 410), which is located on the peripheral surface of the single lumen (112;...; 412) at an angle with the first entry port (104;...; 404) relative to the longitudinal axis, and which is provided for a second guidewire (116; 216; 316; 416) that enters through the second entry port (108;...; 408) and exits from the second exit port (110;...; 410),
wherein:
- the angle between the first entry port (104;...; 404) and the second exit port (110;...; 410) lies from 10° to 180°, and
- the first entry port is located, along the longitudinal axis, between the tip and the at least one second exit port.

2. The catheter (100; 200; 300; 400) as claimed in claim 1, wherein the angle between the first entry port and the second exit port is selected from 30°, 45°, 60°, 90°, 120°, 145°, 150° and 180°, and more specifically, the angle is selected from 90° and 180°.

3. The catheter (100; 200; 300) as claimed in any one of claims 1 or 2, wherein the single lumen (112; 212; 312) has two second exit ports (110, 111; 210, 211; 310, 311), which are located on the peripheral surface of the single lumen at an angle relative to the longitudinal axis, wherein the first entry port is located, along the longitudinal axis, between the tip and the two second exit ports.

4. The catheter (100; 200; 300) as claimed in claim 3, wherein:
- the two second exit ports include a primary second exit port (110; 210; 310) and a secondary second exit port (111; 211; 311),
- the primary second exit port and the secondary second exit port are oppositely located relative to the longitudinal axis and at a distance along the longitudinal axis of the single lumen (112; 212; 312).

5. The catheter (100; 200; 300) as claimed in any one of claims 3 or 4, wherein the angle between the two second exit ports (110, 111; 210, 211; 310, 311) lies from 10° to 180° relative to the longitudinal axis, specifically selected from 30°, 45°, 60°, 90°, 120°, 145°, 150° and 180°, and more specifically, the angle is selected from 90° and 180°.

6. The catheter (100; 200; 300) as claimed in any one of the claims 3 to 5, wherein the angle between the two second exit ports (110, 111; 210, 211; 310, 311) is 180° relative to the longitudinal axis.

7. The catheter (200; 300) as claimed in claim 1, wherein the catheter comprises an expandable balloon (220; 320), which is connected at the distal end of the single lumen (212; 312).

8. The catheter (200; 300) as claimed in claim 7, wherein:
- the expandable balloon (220; 320) is connected to distal end of an inflation lumen (224; 324) and
- a proximal end of the inflation lumen is connected to an inflation port (222; 322) located on the luer (218; 318),
- the inflation lumen is concentric to the single lumen (212; 312).

9. The catheter (200; 300) as claimed in any one of claims 7 or 8, comprising a plurality of joints (234; 334) for enabling an insulated passage across the inflation lumen (224; 324), the plurality of joints (234; 334) being configured for guiding of the first and second guide wires (214, 216; 314, 316) between the single lumen (212; 312) and outside of the catheter (200; 300).

10. The catheter (100; 200; 300) as claimed in any one of claims 3 to 9, wherein:
- the first guidewire (114; 214; 314) enters through the first entry port (104; 204; 304) and exits through the first exit port (106; 206; 306),
- the second guidewire (116; 216; 316) enters through the second entry port (108; 208; 308) and exits either through the primary second exit port (110; 210; 310) or through the secondary second exit port (111; 211; 311).

11. The catheter (300) as claimed in claim 1 or 7, wherein the single lumen (312) is divided in two parts, including a first lumen part (328) and a second lumen part (330), by a dividing wall (332) located longitudinally inside the single lumen (312), wherein:
- the catheter comprises a third entry port (336) located on the luer (318) and connected to the first lumen part (328);
- the second entry port (308) located on the luer (318) is connected to the second lumen part (330); and
- the first entry port (304) is connected to either the first lumen part (328) or the second lumen part (330).

12. The catheter (300) as claimed in claim 11, wherein:
- the first guidewire (314) enters through the first entry port (304) and exits through the first exit port (306),
- the second guidewire (316) enters through the second entry port (308) and exits through the secondary second exit port (311), and
- a third guidewire (326) enters through the third entry port (336) and exits through the primary second exit port (310).

13. The catheter (100; 200; 300; 400) as claimed in any one of claims 1 to 12, wherein the catheter is a micro catheter.

## Patentansprüche

1. Katheter (100; 200; 300; 400) zum Zugang zu einem Lumen, umfassend:
- ein einzelnes Lumen (112; 212; 312; 412) für einen und mehr als einen Führungsdraht, wobei sich das besagte einzelne Lumen (112;212;312;412) entlang einer Längsachse zwischen einem Luer-Anschluss (118; 218; 318; 418) an einem proximalen Ende und einer Spitze (102; 202; 302; 402) an einem distalen Ende erstreckt;
- eine erste Eintrittsöffnung (104; 204; 304; 404), die auf einer Umfangsfläche des einzelnen Lumens (112;...; 412) angeordnet ist, und eine erste Austrittsöffnung (106; 206; 306; 406), die an der Spitze (102;...; 402) angeordnet ist und für einen ersten Führungsdraht (114; 214; 314; 414) vorgesehen ist, der durch die erste Eintrittsöffnung (104;... ; 404) eintritt und aus der ersten Austrittsöffnung (106;...; 406) austritt; und
- eine zweite Eintrittsöffnung (108; 208; 308; 408), die am Luer-Anschluss (118;... ;408) angeordnet ist, und mindestens eine zweite Austrittsöffnung (110; 210; 310; 410), die auf der Umfangsfläche des einzelnen Lumens (112;...; 412) in einem Winkel zu der ersten Eintrittsöffnung (104;...; 404) relativ zu der Längsachse angeordnet ist und für einen zweiten Führungsdraht (116; 216; 316; 416) vorgesehen ist, der durch die zweite Eintrittsöffnung (108;...; 408) eintritt und aus der zweiten Austrittsöffnung (110;...; 410) austritt,
wobei:
- der Winkel zwischen der ersten Eintrittsöffnung (104;...; 404) und der zweiten Austrittsöffnung (110;...; 410) von 10° bis 180° beträgt, und
- die erste Eintrittsöffnung entlang der Längsachse zwischen der Spitze und der mindestens einen zweiten Austrittsöffnung angeordnet ist.

2. Katheter (100; 200; 300; 400) nach Anspruch 1, wobei der Winkel zwischen der ersten Eintrittsöffnung und der zweiten Austrittsöffnung aus 30°, 45°, 60°, 90°, 120°, 145°, 150° und 180° ausgewählt ist, und insbesondere der Winkel aus 90° und 180° ausgewählt ist.

3. Katheter (100; 200; 300) nach einem der Ansprüche 1 oder 2, wobei das einzelne Lumen (112; 212; 312) zwei zweite Austrittsöffnungen (110, 111; 210, 211; 310, 311) aufweist, die auf der Umfangsfläche des einzelnen Lumens in einem Winkel relativ zu der Längsachse angeordnet sind, wobei die erste Eintrittsöffnung entlang der Längsachse zwischen der Spitze und den zweiten Austrittsöffnungen angeordnet ist.

4. Katheter (100; 200; 300) nach Anspruch 3, wobei:
- die zwei zweiten Austrittsöffnungen eine primäre zweite Austrittsöffnung (110; 210; 310) und eine sekundäre zweite Austrittsöffnung (111; 211; 311) umfassen,
- die primäre zweite Austrittsöffnung und die sekundäre zweite Austrittsöffnung entgegengesetzt relativ zu der Längsachse und in einem Abstand entlang der Längsachse des einzelnen Lumens (112; 212; 312) angeordnet sind.

5. Der Katheter (100; 200; 300) nach einem der Ansprüche 3 oder 4, wobei der Winkel zwischen den zwei zweiten Austrittsöffnungen (110, 111; 210, 211; 310, 311) von 10° bis 180° relativ zu der Längsachse beträgt, insbesondere aus 30°, 45°, 60°, 90°, 120°, 145°, 150° und 180° ausgewählt ist, und insbesondere der Winkel aus 90° und 180° ausgewählt ist.

6. Katheter (100; 200; 300) nach einem der Ansprüche 3 bis 5, wobei der Winkel zwischen den zwei zweiten Austrittsöffnungen (110, 111; 210, 211; 310, 311) 180° relativ zu der Längsachse beträgt.

7. Der Katheter (200; 300) nach Anspruch 1, wobei der Katheter einen expandierbaren Ballon (220; 320) umfasst, der am distalen Ende des einzelnen Lumens (212; 312) verbunden ist.

8. Katheter (200; 300) nach Anspruch 7, wobei:
- der expandierbare Ballon (220; 320) mit dem distalen Ende eines Inflationslumens (224; 324) verbunden ist und
- ein proximales Ende des Inflationslumens mit einer am Luer-Anschluss (218; 318) angeordneten Inflationsöffnung (222; 322) verbunden ist,
- das Inflationslumen konzentrisch zu dem einzelnen Lumen (212; 312) ist.

9. Katheter (200; 300) nach einem der Ansprüche 7 oder 8, umfassend eine Vielzahl von Verbindungsstellen (234; 334) zum Ermöglichen eines isolierten Durchgangs durch das Inflationslumen (224; 324), wobei die Vielzahl von Verbindungsstellen (234; 334) dafür konfiguriert ist, den ersten und zweiten Führungsdraht (214, 216; 314, 316) zwischen dem einzelnen Lumen (212; 312) und außerhalb des Katheters (200; 300) zu führen.

10. Katheter (100; 200; 300) nach einem der Ansprüche 3 bis 9, wobei:
- der erste Führungsdraht (114; 214; 314) durch die erste Eintrittsöffnung (104; 204; 304) eintritt und durch die erste Austrittsöffnung (106; 206; 306) austritt,
- der zweite Führungsdraht (116; 216; 316) durch die zweite Eintrittsöffnung (108; 208; 308) eintritt und entweder durch die primäre zweite Austrittsöffnung (110; 210; 310) oder durch die sekundäre zweite Austrittsöffnung (111; 211; 311) austritt.

11. Der Katheter (300) nach Anspruch 1 oder 7, wobei das einzelne Lumen (312) durch eine längs innerhalb des einzelnen Lumens (312) angeordnete Trennwand (332) in zwei Teile unterteilt ist, einschließlich eines ersten Lumenabschnitts (328) und eines zweiten Lumenabschnitts (330), wobei:
- der Katheter eine dritte Eintrittsöffnung (336) umfasst, die am Luer-Anschluss (318) angeordnet und mit dem ersten Lumenabschnitt (328) verbunden ist;
- die am Luer-Anschluss (318) angeordnete zweite Eintrittsöffnung (308) mit dem zweiten Lumenabschnitt (330) verbunden ist; und
- die erste Eintrittsöffnung (304) entweder mit dem ersten Lumenabschnitt (328) oder dem zweiten Lumenabschnitt (330) verbunden ist.

12. Katheter (300) nach Anspruch 11, wobei:
- der erste Führungsdraht (314) durch die erste Eintrittsöffnung (304) eintritt und durch die erste Austrittsöffnung (306) austritt,
- der zweite Führungsdraht (316) durch die zweite Eintrittsöffnung (308) eintritt und durch die sekundäre zweite Austrittsöffnung (311) austritt, und
- ein dritter Führungsdraht (326) durch die dritte Eintrittsöffnung (336) eintritt und durch die primäre zweite Austrittsöffnung (310) austritt.

13. Der Katheter (100; 200; 300; 400) nach einem der Ansprüche 1 bis 12, wobei der Katheter ein Mikrokatheter ist.

## Revendications

1. Un cathéter (100; 200; 300; 400) pour accéder à une lumière, comprenant :
- une lumière unique (112; 212; 312; 412) pour un et plus d'un fil-guide, ladite lumière unique (112;212;312;412) s'étendant le long d'un axe longitudinal entre un raccord Luer (118; 218; 318; 418) à une extrémité proximale et une pointe (102; 202; 302; 402) à une extrémité distale ;
- un premier orifice d'entrée (104; 204; 304; 404), qui est situé sur une surface périphérique de la lumière unique (112;...; 412), et un premier orifice de sortie (106; 206; 306; 406), qui est situé à la pointe (102;...; 402) et qui est prévu pour un premier fil-guide (114; 214; 314; 414) qui entre par le premier orifice d'entrée (104;... ; 404) et sort du premier orifice de sortie (106;...; 406) ; et
- un second orifice d'entrée (108; 208; 308; 408), qui est situé au raccord Luer (118;... ;418), et au moins un second orifice de sortie (110; 210; 310; 410), qui est situé sur la surface périphérique de la lumière unique (112;...; 412) selon un angle par rapport au premier orifice d'entrée (104;...; 404), cet angle étant mesuré par rapport à l'axe longitudinal, et qui est prévu pour un second fil-guide (116; 216; 316; 416) qui entre par le second orifice d'entrée (108;...; 408) et sort du second orifice de sortie (110;...; 410),
dans lequel :
- l'angle entre le premier orifice d'entrée (104;...; 404) et le second orifice de sortie (110;...; 410) est compris entre 10° et 180°, et
- le premier orifice d'entrée est situé, le long de l'axe longitudinal, entre la pointe et au moins un second orifice de sortie.

2. Le cathéter (100; 200; 300; 400) selon la revendication 1, dans lequel l'angle entre le premier orifice d'entrée et le second orifice de sortie est choisi parmi 30°, 45°, 60°, 90°, 120°, 145°, 150° et 180°, et plus spécifiquement, l'angle est choisi parmi 90° et 180°.

3. Le cathéter (100; 200; 300) selon l'une quelconque des revendications 1 ou 2, dans lequel la lumière unique (112; 212; 312) présente deux seconds orifices de sortie (110, 111; 210, 211; 310, 311), qui sont situés sur la surface périphérique de la lumière unique selon un angle par rapport à l'axe longitudinal, dans lequel le premier orifice d'entrée est situé, le long de l'axe longitudinal, entre la pointe et les seconds orifices de sortie.

4. Le cathéter (100; 200; 300) selon la revendication 3, dans lequel :
- les deux seconds orifices de sortie comprennent un orifice de sortie second primaire (110; 210; 310) et un orifice de sortie second secondaire (111; 211; 311),
- l'orifice de sortie primaire du second ensemble et l'orifice de sortie secondaire du second ensemble sont situés de manière opposée par rapport à l'axe longitudinal et à une distance le long de l'axe longitudinal de la lumière unique (112; 212; 312) .

5. Le cathéter (100; 200; 300) selon l'une quelconque des revendications 3 ou 4, dans lequel l'angle entre les deux seconds orifices de sortie (110, 111; 210, 211; 310, 311) est compris entre 10° et 180° par rapport à l'axe longitudinal, spécifiquement choisi parmi 30°, 45°, 60°, 90°, 120°, 145°, 150° et 180°, et plus spécifiquement, l'angle est choisi parmi 90° et 180°.

6. Le cathéter (100; 200; 300) selon l'une quelconque des revendications 3 à 5, dans lequel l'angle entre les deux seconds orifices de sortie (110, 111; 210, 211; 310, 311) est de 180° par rapport à l'axe longitudinal.

7. Le cathéter (200; 300) selon la revendication 1, dans lequel le cathéter comprend un ballonnet expansible (220; 320), qui est raccordé à l'extrémité distale de la lumière unique (212; 312).

8. Le cathéter (200; 300) selon la revendication 7, dans lequel :
- le ballonnet expansible (220; 320) est raccordé à l'extrémité distale d'une lumière de gonflage (224; 324) et
- une extrémité proximale de la lumière de gonflage est raccordée à un orifice de gonflage (222; 322) situé sur le raccord Luer (218; 318),
- la lumière de gonflage est concentrique à la lumière unique (212; 312).

9. Le cathéter (200; 300) selon l'une quelconque des revendications 7 ou 8, comprenant une pluralité de jonctions (234; 334) pour permettre un passage isolé à travers la lumière de gonflage (224; 324), la pluralité de jonctions (234; 334) étant configurée pour guider les premier et second fils-guides (214, 216; 314, 316) entre la lumière unique (212; 312) et l'extérieur du cathéter (200; 300) .

10. Le cathéter (100; 200; 300) selon l'une quelconque des revendications 3 à 9, dans lequel :
- le premier fil-guide (114; 214; 314) entre par le premier orifice d'entrée (104; 204; 304) et sort par le premier orifice de sortie (106; 206; 306),
- le second fil-guide (116; 216; 316) entre par le second orifice d'entrée (108; 208; 308) et sort soit par l'orifice de sortie primaire du second ensemble (110; 210; 310), soit par l'orifice de sortie secondaire du second ensemble (111; 211; 311).

11. Le cathéter (300) selon la revendication 1 ou 7, dans lequel la lumière unique (312) est divisée en deux parties, comprenant une première partie de lumière (328) et une seconde partie de lumière (330), par une paroi de division (332) située longitudinalement à l'intérieur de la lumière unique (312), dans lequel :
- le cathéter comprend un troisième orifice d'entrée (336) situé sur le raccord Luer (318) et raccordé à la première partie de lumière (328) ;
- le second orifice d'entrée (308) situé sur le raccord Luer (318) est raccordé à la seconde partie de lumière (330) ; et
- le premier orifice d'entrée (304) est raccordé soit à la première partie de lumière (328), soit à la seconde partie de lumière (330).

12. Le cathéter (300) selon la revendication 11, dans lequel :
- le premier fil-guide (314) entre par le premier orifice d'entrée (304) et sort par le premier orifice de sortie (306),
- le second fil-guide (316) entre par le second orifice d'entrée (308) et sort par l'orifice de sortie secondaire du second ensemble (311), et
- un troisième fil-guide (326) entre par le troisième orifice d'entrée (336) et sort par l'orifice de sortie primaire du second ensemble (310).

13. Le cathéter (100 ; 200 ; 300 ; 400) selon l'une quelconque des revendications 1 à 12, dans lequel le cathéter est un microcathéter.
